(19) Europäisches Patentamt
European Patent Office
Office européen des brevets

(11) **EP 4 374 780 A1**

(12) **EUROPEAN PATENT APPLICATION**

(43) Date of publication:
**29.05.2024 Bulletin 2024/22**

(21) Application number: **22209785.9**

(22) Date of filing: **28.11.2022**

(51) International Patent Classification (IPC):
**A61B 5/022** (2006.01)   **A61B 5/021** (2006.01)
**A61B 5/0295** (2006.01)   **A61B 5/00** (2006.01)

(52) Cooperative Patent Classification (CPC):
**A61B 5/02233; A61B 5/0024; A61B 5/02125;
A61B 5/0295; A61B 5/7221;** A61B 5/0515;
A61B 2562/0223; A61B 2562/0247; A61B 2562/046

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Koninklijke Philips N.V.
5656 AG Eindhoven (NL)**

(72) Inventors:
• **MUEHLSTEFF, Jens**
  **Eindhoven (NL)**

• **SCHMITT, Lars**
  **Eindhoven (NL)**
• **RAHMER, Juergen Erwin**
  **5656AG Eindhoven (NL)**
• **GLEICH, Bernhard**
  **5656AG Eindhoven (NL)**

(74) Representative: **Philips Intellectual Property &
Standards
High Tech Campus 52
5656 AG Eindhoven (NL)**

(54) **DEVICE FOR USE IN BLOOD PRESSURE MEASUREMENT**

(57) A device for use in blood pressure measurement which includes an inflatable cuff for wrapping around a body part. Wireless pressure sensing functionality is enabled for sensing air pressure inside the inflatable cuff and/or for sensing tissue pressure between the body part surface and the cuff. This is facilitated by use of electromagnetically responsive elements which provide a pressure-dependent electromagnetic response to stimulation by an electromagnetic signal. Each element for example comprises a magnetically responsive element which, responsive to stimulation by a magnetic field, generates a magnetic signal having a characteristic property or feature which varies as a function of pressure applied to the element. For example, a frequency of the magnetic signal response may vary as a function of pressure applied to the element. Through this mechanism, wireless pressure sensing is made possible in the device, through use of transmit/receive electromagnetic coils which generate a stimulation field and sense electromagnetic response signals.

FIG. 2

**Description**

FIELD OF THE INVENTION

The present invention relates to a device for use in blood pressure measurement.

BACKGROUND OF THE INVENTION

Non-invasive blood pressure measurement is traditionally performed using oscillometric techniques utilizing an inflatable cuff which wraps around the arm of a patient and applies a sequence of increasing or decreasing pressures, and senses a pressure response from the arm via an air pressure sensor disposed inside the inflatable bladder of the cuff. The apparatus is known as sphygmomanometer. By performing well-known processing operations upon the pressure readout from the sensor as a function of the applied pressure, a value for diastolic blood pressure and systolic blood pressure can be obtained.

A development in the field of non-invasive blood pressure measurement is described in the document: EP2953528 A1.

This describes a modification of the traditional sphygmomanometer in which an additional pressure sensor is provided, arranged for directly sensing pressure between the patient's body part and the cuff, in addition to the usual air pressure sensor inside the inflatable bladder. This allows a continuous, high fidelity tissue pressure signal readout as a function of time, which allows for more accuracy in computing blood pressure, allows for reading a blood pressure waveform, and allows for deriving other types of hemodynamic parameter.

In preferred examples of this device, an additional shell portion is provided between the tissue pressure sensor and the inflatable bladder to provide a rigid supportive backing for the additional tissue pressure sensor.

In each of these technologies, there are weaknesses in the design of the pressure readout.

In both cases, it is necessary to run a wired connection to the pressure sensor(s) in order to measure the pressure readout. In the case of the traditional sphygmomanometer, this is a communication line into the bladder, or a fluid line to a pressure transducer outside of the bladder. In some examples, the tissue pressure sensor is provided by a fluid-filled pouch which is fluidically coupled to a pressure transducer for reading out a pressure of the fluid in the pouch. Running a fluid line to this fluid filled pouch, through or around the inflatable cuff bladder, is a challenge. These difficulties make current designs mechanically complicated and costly for mass productions (e.g., requiring manual assembly) as well as incurring higher sensitivity to motion artefacts or kink artifacts.

Furthermore, another disadvantage is that at present only a single pressure measurement is possible. This eliminates any possibility of spatially resolved pressure readings, which would be of value in providing more precisely defined clinical measurements. It also imposes a strong sensitivity of measurement to correct placement of the cuff: the single tissue pressure sensor must be placed carefully over the target artery. However, incorporating a plurality of pressure sensors is not practically feasible due to space constraints, and due to the difficulty of running connections to each individual sensor. Too many sensors would interfere with the measurement accuracy.

SUMMARY OF THE INVENTION

Thus, it has been the recognition of the inventors that a variety of challenges and weaknesses exist in the state of the art in this area. These include, by way of summary:

- an inability to derive spatially resolved pressure measurements;
- the need for careful placement of the cuff on the body part to ensure alignment of a tissue pressure sensor with the artery;
- a complex mechanical construction requiring a passage of a fluid or electrical connection from the tissue pressure sensor to the outside of the cuff, resulting in a requirement for manual rather than automated assembly, and resulting in greater sensitivity to artefacts;
- a requirement in some cases for calibration of the tissue pressure sensor before measurement begins;
- no capacity to take account for different arm geometries or physiologies in measurement.

It has been an aim of the inventors to address one or more of the above disadvantages.

The invention is defined by the independent claims. The dependent claims define advantageous embodiments.

According to examples in accordance with an aspect of the invention, there is provided a device for use in blood pressure measurement. The device comprises a cuff for wrapping around a part of a body of a user, and wherein the cuff includes an actuator in the form of an inflatable bladder for use in changing a pressure applied to the body part by the cuff. The device may include a cuff pressure sensor arranged for sensing a gas pressure inside the inflatable bladder. Additionally or alternatively, the device may include a tissue pressure sensor arranged for sensing a pressure between a surface of the body part and the cuff.

The cuff pressure sensor and/or the tissue pressure sensor comprises: one or more magnetically responsive elements arranged so as to be in pressure-coupling arrangement with the user's body part when the cuff is worn, so that changes in relative pressure between the cuff and the body part during use are coupled to a surface of each magnetically responsive element. Each of the one or more magnetically responsive elements is adapted to be magnetically stimulated upon exposure to a magnetic field and to generate a magnetic signal responsive thereto, and wherein the magnetically responsive element is such that said magnetic signal varies as a function of applied pressure on the magnetically responsive element.

The device further includes one or more field generator coils for generating a magnetic field across a volume, wherein the one or more magnetically responsive elements are located within said volume.

The device further includes a receiver coil arrangement comprising one or more field receiver coils for sampling said magnetic field across a sampling space within the volume.

The device further includes a processing unit comprising one or more processors, the processing unit including: a driver module for driving the generator coils to generate the magnetic field, and a pressure readout module arranged to detect applied pressure at each magnetically responsive element based on sensing the magnetic signals from the elements within the sampled magnetic field.

The proposed concept is to use electromagnetically responsive/sensitive elements which exhibit a pressure-dependent electromagnetic response to electromagnetic stimulation to provide wireless pressure sensing at the site of each element. These sensitive elements may be facilitated by magnetic or magnetizable bodies which exhibit the mentioned electromagnetically responsive properties, e.g., as described in the document WO 2011/30249 A1. Additionally, or alternatively, they may be facilitated by magneto-mechanical oscillators, e.g., as described in the document US 2020/397320 A1.

The technical advantage of this is to permit wireless readout of actuator (bladder) pressure and/or tissue pressure, which allows for improved structural simplicity since a fluid or communication line does not need to be run through the inflatable bladder of the cuff.

In some cases, the tissue pressure sensor has at least a portion arranged to be mechanically coupled by the cuff against the body part when the cuff is in use.

Each magnetically responsive element has a physical property which varies as a function of pressure to which it is exposed, such that the stimulated magnetic signal response from the element varies with pressure.

In some embodiments, this may arise through providing each element such that it comprises a magnetic or magnetizable body and wherein a magnetization state of the body varies as a function of applied pressure, for example due to shape deformation, such that the magnetic signal varies. In other embodiments, it may arise through providing each element such that it comprises at least a component which exhibits an oscillation responsive to an applied oscillating magnetic field, and wherein an oscillation response varies as a function of applied pressure. This could be facilitated with the magnetizable bodies or through provision of a magneto-mechanical oscillator which has a magnetic object disposed inside a pressurized casing.

In terms of the physical principle underlying the wireless pressure readout, the following optional details may be considered.

In some embodiments, each of the one or more magnetically responsive elements may comprise a magnetic or magnetizable body which is deformable and exhibits a deformation which varies as a function of applied pressure, and wherein the magnetic signal generated by each body varies as a function of the deformation state of the body.

In some embodiments, the driver module is adapted to drive generation of an oscillating magnetic field.

In some embodiments, each of the magnetically responsive elements comprises at least a part which is adapted to mechanically oscillate at a respective characteristic resonant frequency in response to exposure to the oscillating magnetic field, said oscillation generating the magnetic signal for the respective element.

In some embodiments, each of the magnetically responsive elements is such that said characteristic resonant frequency varies within a respective characteristic resonant frequency band/range as a function of applied pressure on the element, i.e., the resonant frequency changes as a function of pressure on the magnetically responsive element with a known relationship. Thus, pressure can be sensed by sensing changes in the magnetic signal frequency originating from the element and using for instance a pre-determined transfer function or lookup table to map the sensed return frequency of the returned magnetic signal to a pressure readout.

In some embodiments, the device comprises a plurality of the magnetically responsive elements.

In some embodiments, each magnetically responsive element is adapted to oscillate with a characteristic resonant frequency which is different from every other element; and which varies as a function of applied pressure within a respective characteristic resonant frequency band/range which is different from every other element. In other words, the magnetic resonant frequency of each element varies across a defined range as a function of applied pressure in accordance with a predefined (and known) relationship, and wherein this respective variation range is different for each respective magnetically responsive element compared to every other magnetically responsive element. This allows for distinguishing magnetic signals originating from different of the magnetically responsive elements. The bands/ranges may preferably be non-overlapping.

For each receiver coil signal, the signal in the frequency domain can be analyzed to detect variations as a function of time within each known frequency band, and from this, pressure can be detected for each separate magnetically responsive element based on a respective transfer function or lookup table mapping measured frequency to pressure measurement.

Furthermore, localization of each individual localizable element is possible if the receiver coil arrangement comprises a plurality of receiver coils, by triangulating the signal strength at different receiver coils within the characteristic resonant frequency band for the different coils.

In some embodiments, the tissue pressure sensor comprises a plurality of the magnetically responsive elements, arranged to be sensitive to pressure at different locations across a surface of the body part of the user during use.

In some embodiments, the readout module is adapted to detect a pressure reading for each magnetically responsive element.

In some embodiments, the readout module is adapted to compute a pressure distribution across a surface of the body part based on a known spatial arrangement of the magnetically responsive elements relative to the cuff.

In some embodiments, the receiver coil arrangement comprises a plurality of receiver coils.

In some embodiments, the processor is adapted to determine a positioning of each of the one or more magnetically responsive elements within the volume based on analysis of a signal strength of the magnetic signal from each element sensed at each receiver coil.

In some embodiments, the device includes a plurality of the magnetically responsive elements arranged for sensing pressure at different locations across a surface of the body part when the cuff is worn. In some embodiments, the processor is adapted to determine geometry or shape information related to the body part based on the detected positioning of the plurality of elements, e.g. arm circumference.

In some embodiments, the receiver coil arrangement and/or the generator coils may be included in a separate unit from the cuff, optionally attachable to an outside of the cuff or attachable to a part of the body. This permits detection of position or orientation information of the magnetically responsive elements relative to a fixed reference frame defined by the separate unit comprising the coils as opposed to the reference frame of the cuff itself.

In some embodiments, the device includes a plurality of the magnetically responsive elements arranged for sensing pressure at different locations across a surface of the body part when the cuff is worn, and wherein the processing unit is configured to perform one or more of the following steps: detect the magnetic signal associated with each different magnetically responsive element; determine a signal strength associated with each magnetic signal; identify the signal with the highest signal strength; compute a pressure reading based on the identified magnetic signal; and optionally forward the identified pressure reading to a biological parameter measurement module.

Thus, in this set of embodiments, a highest quality signal is identified (e.g. highest SNR), and this is used to provide a single pressure measurement.

In some embodiments, the device includes a plurality of the magnetically responsive elements arranged for sensing pressure at different locations across a surface of the body part when the cuff is worn; and wherein the processing unit is adapted to perform one or more of the following steps: detect the magnetic signal associated with each different element; determine a signal strength associated with each magnetic signal; determine a pressure measurement for each element based on the respective magnetic signal; generate a weighted average pressure reading, wherein the weightings are determined based on the signal strength of the magnetic signal associated with each respective pressure measurement; and optionally forward the weighted average pressure reading to a biological parameter measurement module. Thus, here, rather than discard all signals apart from the one signal with the highest strength, a composite signal is formed from a combination of the plurality of signals, weighted according to signal strength.

If the generated field is an oscillating field, the signal strength may correspond to an amplitude of the signal.

A signal strength may correspond to a signal to noise ratio.

In some embodiments, the tissue pressure sensor comprises a plurality of the magnetically responsive elements, arranged to be sensitive to pressure at different locations across a surface of the body part of the user during use; and wherein the plurality of bodies is arranged in a regular two-dimensional array formation having an array area which extends across a tissue-facing area of the cuff.

In some embodiments, the tissue pressure sensor comprises a plurality of the magnetically responsive elements, arranged to be sensitive to pressure at different locations across a surface of the body part of the user during use, and wherein, when the cuff is wrapped around the body part, the body part extends through the cuff between a distal and proximal end of the cuff, and wherein the plurality of elements includes a first element closer to the proximal end, and a second element closer to the distal end. This permits, according to some embodiments, for measurement of flow or pulse wave characteristics. For example, in some embodiments, the processing unit is adapted to determine a pulse transit time based on pressure readings from the first and second magnetically responsive elements.

In some embodiments, the tissue pressure sensor comprises a plurality of the magnetically responsive elements, arranged to be sensitive to pressure at different locations across a surface of the body part of the user during use; wherein the plurality of magnetically responsive elements is arranged such that, when the cuff is wrapped around the body part, the elements are at different circumferential locations around the body part, i.e. located at different locations around the inner circumference of the cuff. This reduces rotational sensitivity of the pressure measurements.

In some embodiments, the processing unit is adapted to determine an orientation state of the cuff based on the signals from the plurality of magnetically responsive elements. Optionally, in this case, the receiver coil arrangement and/or the generator coils may be included in a separate unit from the cuff, optionally attachable to an outside of the cuff or attachable to a part of the body. The separate unit could be adapted to be fixable by a user to the cuff or the body in a defined orientation state relative to the body. This thereby provides a fixed rotational anchor point relative to which the orientation state of the cuff can be determined. This is just one example for how to implement this example feature.

In some embodiments, the plurality of elements includes an annular or arcuate array of elements arranged, when the cuff is wrapped around the body part, such that the array extends around the whole or part of the circumference of the body part, i.e. the inner circumference of the cuff.

In this embodiment, the processing unit is preferably adapted to select a pressure reading from the set of pressure readings having a highest signal strength and forward the selected signal to a biological parameter measurement module. Thus, this effectively allows the cuff to be applied in any orientation, and the sensing element positioned closest to the artery is automatically detected based on analysis of signal strength.

In some embodiments, each magnetically responsive element is located inside a respective fluid-filled pocket. Pressure applied to the fluid-filled pocket is thus efficiently transferred onto an outer surface of the magnetically responsive element.

In some embodiments, the one or more processors further include a biological parameter measurement module including one or more algorithms for computing one or more biological parameters based on the one or more pressure readings.

In some embodiments, the device includes a shell portion, wherein the shell portion is arranged so as to be located between the actuator and the body part when the cuff is wrapped around the body part. The shell portion may be arranged to surround the body part when the cuff is wrapped around the body part. The shell portion may comprise a plurality of overlapping sections configured to slide relatively to each other, permitting variation of the diameter of the shell portion responsive to application of pressure by the actuator.

The shell-portion is preferably kinking-proof shell. More details can be found in the document EP2953528 A1.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 shows an example hemodynamic parameter measurement apparatus comprising an inflatable cuff;
Fig. 2 schematically illustrates an example device in accordance with one or more embodiments of the invention;
Fig. 3 shows a first example magnetically responsive element in accordance with one or more embodiments;
Fig. 4 schematically illustrates components of an example processing unit including a driver module and pressure readout module, suitable for use with a device in accordance with one or more embodiments;
Figs. 5-6 illustrate a further example magnetically responsive element in accordance with one or more embodiments;
Fig. 7 schematically illustrates components of an example processing unit including a driver module and pressure readout module, suitable for use with a device in accordance with one or more embodiments;
Fig. 8 illustrates an example pressure sensor for use in a device according to one or more embodiments;
Fig. 9 illustrates measurement of pulse transit time using magnetically responsive elements according to one or more embodiments;
Fig. 10 illustrates use of a plurality of magnetically responsive elements for measuring arm circumference;
Fig. 11 illustrates use of an annular array of magnetically responsive elements to achieve a rotationally insensitive device; and
Fig. 12 illustrates a block diagram of an example device according to one or more embodiments of the invention.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures. are merely schematic and are not drawn to scale. It should also be understood that the same reference numerals are used throughout the Figures. to indicate the same or similar parts.

The invention provides a device for use in blood pressure measurement which includes an inflatable cuff for wrapping around a body part. Wireless pressure sensing functionality is enabled for sensing air pressure inside the inflatable cuff and/or for sensing tissue pressure between the body part surface and the cuff. This is facilitated by use of electromagnetically responsive elements which provide a pressure-dependent electromagnetic response to stimulation by an electromagnetic signal. Each element for example comprises a magnetically responsive element which, responsive to stimulation by a magnetic field, generates a magnetic signal having a characteristic property or feature which varies as a function of pressure applied to the body. For example, a frequency of the magnetic signal response may vary as a function of pressure applied to the body. Through this mechanism, wireless pressure sensing is made possible in the device, through use of transmit/receive electromagnetic coils which generate a stimulation field and sense electromagnetic response signals.

If more than one of the magnetically responsive bodies is included, then spatially resolvable pressure sensing is possible and/or improved signal quality of a single pressure measurement is possible.

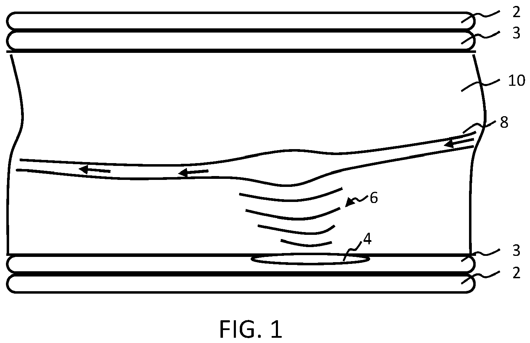

By way of background, Fig. 1 shows a schematic illustration of a prior art cuff-based hemodynamic parameter measurement apparatus to which embodiments of the present invention might be applied to provide an inventive development. The embodiments of the invention are not restricted for their effect by application to this example prior art device and can be applied more generally a wide variety of different devices.

The apparatus shown in Fig. 1 can be used to detect blood pressure and/or other hemodynamic parameter measurements such as cardiac output or stroke volume. The illustration shows the cuff applied to the upper arm 10 of a patient. An artery 8 of the patient is schematically shown. The apparatus has a design which differs from the most standard cuff-based blood pressure measurement apparatuses in that it includes a dedicated tissue pressure sensor 4 which the cuff acts to hold against the tissue of the skin when the cuff is inflated. The cuff includes a pneumatic actuator in the form of an inflatable bladder 2 for use in changing a pressure applied to the body part 10 by the cuff. The tissue pressure sensor 4 is arranged for sensing a pressure between a surface of the user's body part and the cuff. Independently of the tissue pressure sensor, one or more operational parameters of the pneumatic actuator can be sampled. For example, a pressure inside the inflatable bladder can be sensed. An actuator activity signal indicative of a pumping power level or pumping rate of a pump of the pneumatic actuator can also be sampled.

Standard blood pressure measurement cuffs use the bladder air pressure as the sole measurement signal for sensing blood pressure. By utilizing a tissue pressure sensor 4, the apparatus shown in Fig. 1 can achieve superior quality results and also enables the measurement of advanced hemodynamic parameters such as stroke volume, cardiac output and fluid responsiveness in addition to blood pressure. In particular, in standard air blood pressure cuffs, dampening air is assumed to destroy more than 90% of the amplitude and contour of a tissue pressure pulse wave. By contrast, by using a dedicated tissue pressure sensor, the design illustrated in Fig. 1 allows recording high-fidelity (HiFi) artery blood pressure and pulse waveform.

The tissue pressure sensor 4 may be implemented as, e.g. a fluid-filled bag which provides a conforming layer of fluid between the cuff bladder 2 and the surface of the user's body. In this way, the integrated pneumatic actuator enables hydraulic coupling of the tissue pressure sensor 4 to the rigid shell-encased upper arm tissue. When blood pulses in the artery, this results in a pressure wave 6 which can be detected by the tissue pressure sensor 4. The tissue pressure sensor 4 can be connected to a pressure transducer via a fluid-filled tube/line. The pressure transducer converts the pressure in the fluid into an electrical signal.

The operating principle is based on coupling of the pressure sensor to the body part (e.g. arm) in order to transcutaneously record tissue pressure pulse waves that result from arterial pulsation (e.g. brachial artery). In similar fashion to a conventional upper arm air blood pressure cuff, the cuff compresses the upper arm using an integrated pneumatic actuator with rising clamping pressure. However, the actual compression may be achieved via the narrowing diameter of a rigid circumferential shell.

In the example illustrated in Fig. 1, the cuff includes a shell portion 3, wherein the shell portion is arranged so as to be located between the pneumatic actuator 2 and the body part when the cuff is worn on the body part, and arranged to surround the body part when the cuff is worn. The tissue pressure sensor 4 is arranged so as to be positioned between the shell 3 and the body part when the device is worn on the body part. The shell structure may be relatively stiff. Consequently, the measurement accuracy can be improved, since the tissue pressure 4 is measured against a relatively stiff support, which prevents dampening of the amplitude and shape of the signal. In particular, if a tissue pressure sensor unit 4 is located at least partially between the shell portion 3 and the body part, a high accuracy of the signals measured by the tissue pressure sensor unit can be achieved. With such a configuration of the tissue pressure sensor unit, the shell structure does not absorb or attenuate the arterial pressure signal.

The shell portion 3 may include overlapping sections, and wherein the overlapping sections of the shell portion may move or slide relatively to each other, thereby reducing the diameter of the shell portion responsive to increasing applied pressure by the pneumatic actuator.

If the tissue pressure sensor uses a fluid-based sensing mechanism, as described above, then an initial calibration may be needed in which the pressure transducer, to which the fluid filled pad 4 of the pressure sensor is fluidically coupled, is exposed to atmospheric pressure prior to beginning measurement.

The design described above permits non-invasive hemodynamic monitoring and enables measurement of blood pressure, as well as cardiac output and other hemodynamic parameters.

For more expansive details about this example hemodynamic parameter measurement apparatus, reference is made to the document EP2953528 A1 which describes the cuff design in more detail.

The device according to some embodiments of the present invention may include some or all of the components of the hemodynamic parameter measuring system described in the document EP2953528 A1.

As briefly explained above, embodiments of the present invention propose to use pressure-dependent electromagnetic transducers to enable wireless pressure readout. Such a solution also permits measurement of pressure spatial distribution.

Various embodiments of the invention may provide some or all of the following advantages:

Measurement of spatially resolved pressures of an area of an underlying artery.
Reduced sensitivity to artifacts.
Reduced sensitivity of measurement to rotational position of the cuff relative to the body part.
Simplified method of use by a user (e.g. since less accuracy is required in cuff placement).
Eliminating a need for calibration of a fluid-based sensing means (e.g., no manual zeroing procedure required before first measurement).
Automatically determining optimal pressure signals for parameter interference.
Structurally simpler construction.
Access to new parameters such as pulse transit time.

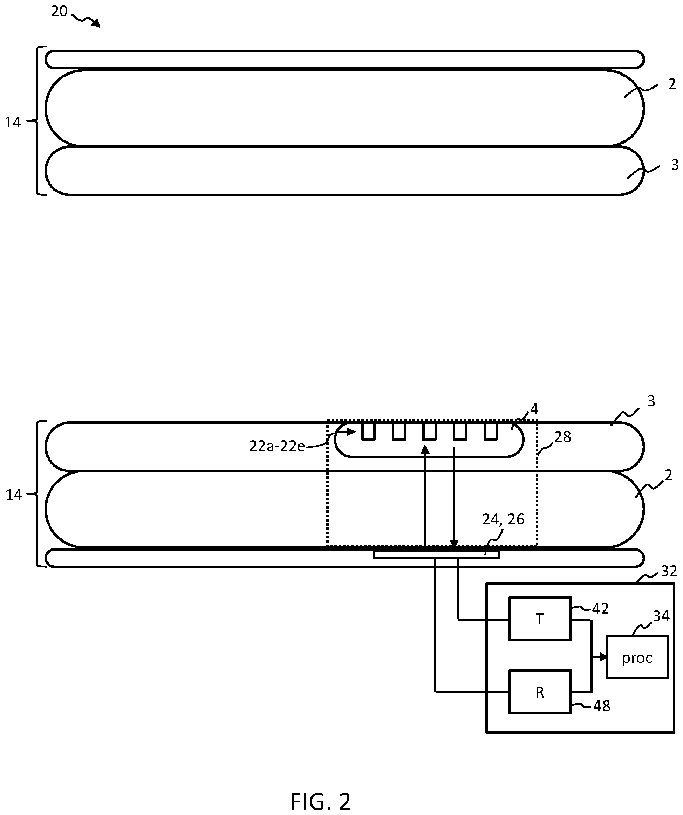

Fig. 2 schematically illustrates an example device 20 in accordance with one or more embodiments of the present invention.

The device 20 is for use in blood pressure measurement and/or measurement of other hemodynamic parameters.

The device 20 includes a cuff 14 for wrapping around a part of a body of a user, and wherein the cuff includes an actuator in the form of an inflatable bladder 2 for use in changing a pressure applied to the body part by the cuff. Fig. 2 shows a cross-sectional view of the cuff in a position wrapped around a body part (not shown), along a plane parallel with a longitudinal axis and radial axis of the cuff, and perpendicular to a circumferential axis of the device. Thus, Fig. 2 shows a slice through the layers forming the cuff. The layers in Fig. 2 are not shown to scale but are enlarged to enable easier viewing of the structure and components.

In the illustrated example, the device 20 includes a tissue pressure sensor 4 arranged for sensing a pressure between a surface of the body part and the cuff 14. Additionally, or alternatively, the device may include a cuff pressure sensor arranged for sensing a gas pressure inside the inflatable bladder.

The tissue pressure sensor 4 in this example comprises one or more magnetically responsive elements 22 arranged so as to be in pressure coupling arrangement with the user's body part when the cuff is worn, so that changes in relative pressure between the cuff 14 and the body part during use are coupled to a surface of each magnetically responsive element. In alternative embodiments, the magnetically responsive elements might alternatively or additionally be comprised by the aforementioned bladder air pressure sensor.

Each of the one or more magnetically responsive elements 22 is adapted to be magnetically stimulated upon exposure to a magnetic field and to generate a magnetic signal responsive thereto, and wherein each magnetically responsive element is such that said magnetic signal varies as a function of applied pressure on the magnetically responsive element.

The device further includes one or more field generator coils 24 (otherwise known as 'transmit coils') for generating a magnetic field across a volume 28, wherein the one or more magnetically responsive elements are located within said volume.

The device further includes a receiver coil arrangement 26 comprising one or more field receiver coils (otherwise known as 'receive coils') for sampling said magnetic field across a sampling space within the volume. In the example of Fig. 2, the field generator coils and receiver coil arrangement 26 are shown as comprised by a single coil unit. For example, the transmit coils and receive coils might be provided in an interleaved array pattern, or the receive and transmit coils might be positioned spaced from one another in a circumferential direction (into the page from the perspective of Fig. 2). The transmit and receive coils could also be provided by a same set of physical coils which are alternated between transmit and receive modes in a duty cycle operation. There may be just one transmit coil and one receive coil or there may be a plurality of each of the transmit or receive coils.

The device further comprises a processing unit 32 comprising one or more processors 34. The processing unit includes: a driver module 42 (or transmit module) for driving the generator coils to generate the magnetic field; and a pressure readout module 48 arranged to detect applied pressure at each magnetically responsive element based on sensing the magnetic signals from the elements within the sampled magnetic field. Although the driver module 42 and readout module 48 are shown as separate components, they might both be provided by a single transmit/receive (T/R) module in some embodiments. Furthermore, although the driver 42 and readout 48 modules are shown as separate hardware elements, in further embodiments, their functionality might be provided by software modules of a single processing component.

In the example of Fig. 2, the processing unit 32 further comprises one or more processors 34. In some embodiments, the one or more processors 34 might further include a biological parameter measurement module including one or more algorithms for computing one or more biological parameters based on the one or more pressure readings. The one or more processors might be configured to process the pressure signals output from the readout module to derive a measure of blood pressure and/or one or more further hemodynamic parameters. One suitable example method for doing this is described in the document US 2015/359442. A further suitable example method for doing this is described in the document US 2015/320364. In each of these methods, the tissue pressure sensor of the system of the present invention may be used to measure the pressure pulse signals referred to in the referenced documents.

Optionally, and as illustrated in Fig. 2, the cuff 14 can include a shell portion 3 located between the inflatable bladder 2 and the body part when the device is worn and having at least a portion located between the tissue pressure sensor 4 and the inflatable bladder 2 when the device is worn. The shell portion is arranged to surround the body part when the cuff is wrapped around the body part. The shell portion may comprise a plurality of overlapping sections configured to slide relatively to each other, permitting variation of the diameter of the shell portion responsive to application of pressure by the actuator. The structure and design of the shell portion is described in more detail in the document EP2953528 A1 but wherein the pressure sensor unit with gel cushion (labelled 18 in EP2953528 A1) is replaced by the tissue pressure sensor 4 according to any of the embodiments outlined in this document.

As explained in paragraph 59 of EP2953528A1 (to which the reader is referred), the shell portion 3 may be made from a plastic material, such as polyethylene. The thickness of the shell may be chosen so that the shell does not buckle when pressure is applied to the inflatable bladder 2, while at the same time the shell is flexible enough to allow for a certain deformation of the shell 20. That is, when pressure is applied to the bladder, overlapping edge regions of the shell may move or slide relatively to each other so as to reduce the diameter of the shell. However, the shell thereby remains substantially ringshaped. Due to the provision of the shell 3, the formation of wrinkles or kinks in the compression-acting surface of the cuff 14 can be significantly reduced. Therefore, subcutaneous bleeding at the measuring site can be avoided and the measurement accuracy is significantly improved.

As outlined above, embodiments of the invention propose use of wireless-readout pressure sensing elements which each comprise a magnetically responsive element 22 which elicits a pressure-dependent magnetic response in the presence of stimulating magnetic field. Each element comprises a magnetically responsive element 22. In the illustration of Fig. 2, a set of five magnetically responsive elements are shown for purposes of illustration, but more or fewer could be provided. At minimum, one should be provided.

There are different ways of implementing the magnetically responsive elements 22.

One approach is described in the document WO 2011/030249A1. This employs use of ferromagnetic bodies which have a magnetization state which varies as a function of a pressure applied to the bodies. This can lead to a change in oscillation frequency of the body when exposed to an oscillating magnetic field, allowing pressure to be transduced by detecting a frequency of the magnetic response signal generated by the body.

Another approach is described in the document US 2020/397320 A1. This employs use of magneto-mechanical oscillators. Here, each magnetically responsive element comprises a casing with a diffusion blocking layer for maintaining a predetermined pressure within the casing and a magneto-mechanical oscillator with a magnetic object providing a permanent magnetic moment. The magneto-mechanical oscillator transduces an external magnetic or electromagnetic excitation field into a mechanical oscillation of the magnetic object, wherein at least a part of the casing is flexible for allowing to transduce external pressure changes into changes of the mechanical oscillation of the magnetic object.

Full implementation details of the technology is described for example in the documents WO 2011/030249A1 and US 2020/397320 A1.

This technology enables very small transducers 22 (in the order of a few millimeters in size), which permit measurement of pressure, temperature or orientation. The readout from the transducer can be achieved wirelessly with no disturbances of the measured object. Due to the small form factor and wireless readout, multiple transducers can be incorporated in an object, which allows the observation of distributions of a parameter of interest in a spatial region.

A summary of relevant implementation details will now be outlined, although the reader is referred to any of the documents US 2020/397320 A1 and WO 2011/030249 A1 for more expansive details.

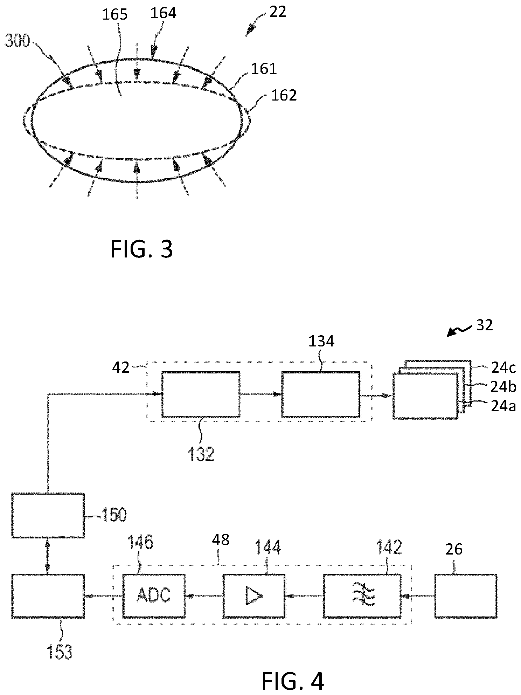

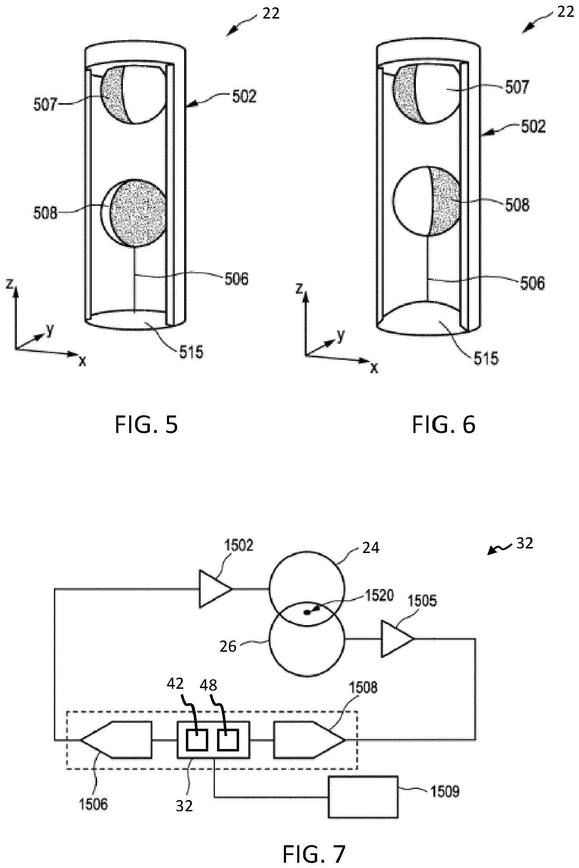

In accordance with a first approach described in WO 2011/030249 A1, we refer in particular to Figs. 6-10 and page 27, line 19 to page 29, line 30 which describes examples of the magnetically responsive elements 22 (referred to as "magnetic pressure measurement devices 60, 70") and their structural configuration. Figs. 4 and 5 and page 22, line 12 to page 25, line 29 describe the implementation of the field generation and field sensing for purposes of obtaining pressure measurements.

Figs. 3 shows an example of the magnetically responsive element 22. The magnetically responsive element in this case is a deformable ferromagnetic body, in particular a hollow, substantially ellipsoidal or spherical- like body.

The receiver coil arrangement 26 and pressure readout module 42 are adapted to acquire magnetic detection signals from the element 22 which depend on the changes of the physical properties of said magnetically responsive element 22 caused by applied pressure on the element.

In operation, a magnetic field, generally a magnetic RF field (an oscillating magnetic field), is generated by the driver module 42 and field generator coils 24 in a volume 28 containing the magnetically responsive element 22. At least one receiving coil 26 is adapted for sampling the magnetic field and detecting therein magnetic signals which have been generated by the magnetically responsive element in response to the generated field. The magnetically responsive element 22 might be understood or modelled as a small ferromagnetic rod or disc which changes its physical properties due to pressure. If a pressure is applied to the ferromagnetic rod, the rod changes its physical properties so that also the magnetic signals which are acquired by the receiver coil arrangement 26 change. These changes of the detected magnetic signals particularly depend on the changes in magnetization of the magnetically responsive element 22 and on the changes of the physical properties of the magnetically responsive element 22. Using the pressure readout module 48, the pressure being exerted on the magnetically responsive element 22 can be inferred from the evaluation of the change of the detected magnetic signals. In case that the field generated by the field generator coils (which might be termed the stimulation field) is a sinusoidal field (i.e. oscillatory field or RF field) the pressure readout can be evaluated by comparing the harmonic frequency of the detected magnetic signals from the element 22 with the harmonic frequency of the originally generated field. The change in the harmonic frequency is a function of a characteristic resonant frequency of the magnetically responsive element 22, wherein this frequency varies as a function of applied pressure. Thus, detecting changes in dominant frequency components in the sampled field can be used to determine pressure changes on the element.

The pressure can be measured as a function of time. The pressure can also be measured as a function of space, if several magnetically responsive elements 22 are employed.

In some embodiments, the magnetic signals generated by the magnetically responsive element 22 varies due to changes in shape of the magnetically responsive element 22. If the magnetically responsive element 22 is made of a deformable material, the shape of the magnetically responsive element 22 changes due to the applied pressure on the magnetically responsive element 22. This pressure-induced shape change can be mechanically modeled so that by comparing the magnetic signal response with the applied magnetic signal, the pressure can be calculated according to the mechanical model. The difference between the applied and the detected magnetic signals then result from a change in the magnetic anisotropy of the magnetically responsive element 22.

Therefore, in some embodiments, the pressure readout module may be configured to evaluate the change of the magnetic anisotropy of the magnetically responsive element 22 caused by the change of the shape of the magnetically responsive element 22. This can be done explicitly or could be achieved in practice through use of a pre-determined lookup table or transfer function computed in advance based on such a model. For example, if the magnetically responsive element 22 has for example an ellipsoidal shape, the element becomes flatter whereby the magnetic anisotropy is increased. The change in anisotropy then results in a shift of the harmonics of the magnetic signal which is a reliable quantity for measuring the internal pressure of the examination object. More precisely, the shift of the harmonics of the magnetic signal is caused by the change of the magnetization curve (B-H curve) of the magnetically responsive element 22 which itself is caused by the shape change of the device.

In order to receive reliable measurement results it is therefore desirable that the magnetically responsive element 22 has an elongated shape since the demagnetization factor is smaller for elongated shapes so that the B-H curve is steeper and the magnetically responsive element 22 is saturated faster. In that case the magnetic pressure measurement device can be brought to the state of saturation even at a relatively small field strength of less than 3 mT.

According to a further one or more embodiments, the pressure readout module 48 may be adapted to evaluate the change of the magnetic resonance of the magnetically responsive element 22 caused by the change of the shape of the magnetically responsive element 22. In this set of embodiments, the applied magnetic field excites the magnetically responsive element 22 so that the device is forced to mechanically oscillate. The magnetically responsive element 22 therefore oscillates in such a way that its shape is compressed and released continuously over time due to the magnetic excitation. Due to the pressure-induced shape change a shift of the resonance frequencies of the oscillation of the magnetically responsive element 22 occurs. Similar to the above-mentioned embodiment, this shift of the resonance frequencies has an influence on the magnetic detection signals which are acquired by the receiver coils 26. Therefore, it is possible to determine the pressure experienced by the magnetically responsive element 22 by evaluation of the change of the frequency of the magnetic detection signals using the pressure readout module 48.

Figs. 3 shows an example of the magnetically responsive element 22 according to the above-outlined approach. In this figure a sample pressure applied onto the magnetically responsive element is indicated by dashed arrows.

The magnetically responsive element 22 is preferably made of a pure iron mantel 164 which is filled with gas 165. The magnetically responsive element 22 is therefore deformable. During the measurement, the magnetically responsive element 22 changes its shape due to the applied pressure 300. If the pressure 300 is increased, magnetically responsive element 22 is compressed like a balloon from an expanded state 161 to a compressed state 162. The B-H curve of the magnetically responsive element 22 changes, respectively gets steeper, when the pressure 300 is increased and the element 60 is compressed 62. Furthermore, it is preferred that the magnetic pressure measurement device 60 has an elongated shape, in particular an ellipsoidal shape in order to have a considerably high magnetic anisotropy. It has to be noted that of course other shapes (e.g. a spherical shape) than the one indicated are possible. By example, the length for the magnetic pressure measurement device 60 is preferably in the range of about 50 $\mu m$.

A magnetic field generated by the magnetic field generator coils 24 influences the magnetization of the magnetically responsive element 22.

Fig. 4 shows an example configuration for the magnetic field generator coils 23, the receiver coil arrangement 26, the driver module 42 and the pressure readout module 48.

In this example, the one or more field generator coils 24 comprise a set of three pairs 24a, 24b, 24c of oppositely arranged magnetic coil elements. These magnetic field coils 24a, 24b, 24c are controlled by a driver module 42, preferably comprising a separate magnetic field signal generation subunit for each coil element (or at least each pair of coil elements) of said set of magnetic field coils 24a, 24b, 24c. Said magnetic field driver module 42 comprises a magnetic field current source 132 (preferably including a current amplifier) and filter unit 134 for providing a magnetic field current to the respective magnetic field coils 24a, 24b, 24c. The magnetic field current source 132 is adapted for generating an AC current and is also controlled by a control unit 150.

The above-described magnetic field generating means are, as already mentioned, adapted for generating a magnetic field in order to influence the magnetization of the example magnetically responsive element 22. The receiver coil arrangement 26 is adapted to detect the magnetic response signals which are caused by the induced changes of the magnetization of the element 22. As discussed above, these magnetic signals furthermore depend on the changes of the physical properties of the magnetically responsive element 22 which are caused by applied pressure. In some embodiments, the applied pressure causes a change in resonant frequency of the element 22 when exposed to an oscillating magnetic field.

According to the particular example of Fig. 3 and Fig. 4, these changes of the physical properties in effect mean a change of the shape of the magnetically responsive element 22 which occur due to the applied pressure on the element. In other words this means that, due to the deformable characteristics of the magnetically responsive element 22 the magnetization curve (the B-H curve) of the magnetically responsive element 22 is changed due to the pressure which has an influence on the acquired magnetic response signals. In particular, the demagnetization factor is reduced if the magnetically responsive element 22 is compressed and therefore elongated so that thereby the steepness of the B-H curve is increased.

In the following, the arrangement of the receiver coil arrangement 26 and the pressure readout module 48 within the processing unit will be explained.

The pressure readout module 48 receives magnetic signals detected by the receiver coil arrangement 26. Said pressure readout module 48 comprises a filter unit 142 for filtering the received detection signals. The aim of this filtering is to separate measured values from other, interfering signals. To this end, the filter unit 142 may be designed for example such that signals which have temporal frequencies that are smaller than the temporal frequencies with which the receiver coils 26 are operated, or which are above these temporal frequencies, do not pass the filter unit 142. The filter unit might also be used to separate signals of different frequency ranges in order to distinguish signals from different magnetically responsive elements, as mentioned above. These signals are then transmitted via an amplifier unit 144 to an analog/digital converter 146 (ADC). The digitalized signals produced by the ADC 146 are finally lead to an evaluation means 153, which evaluates the detection signals in order to determine the applied pressure.

These evaluation means 153 evaluate the changes of the magnetic anisotropy of the magnetically responsive element 22 which are caused by the pressure-induced shape changes of the magnetically responsive element 22. The applied pressure can thereby be determined for example using a mechanical modeling of the magnetically responsive element 22. In practice, a transfer function or lookup table may be used to convert one or more properties of the measured magnetic signals into pressure measurement readouts. In order to derive such a transfer function or lookup table, the B-H curve of the magnetically responsive element 22 might be measured for different pressures when a magnetic field as the one mentioned above is applied.

Furthermore, as can be seen from Fig. 4 a control unit 150 may additionally be provided which is adapted for controlling the driver module to generate and provide control currents to the respective field generator coils 24a, 24b, 24c to generate the above-mentioned magnetic field for influencing the magnetization of the magnetically responsive element 22. The control unit may be comprised by or fulfilled by the one or more processors 34 mentioned previously. Since the characteristics of the magnetic field, especially the frequency of the applied magnetic field, needs to be known for the evaluation respectively the determination of the pressure, the control unit 150 is also connected to the evaluation means 153.

Fig. 3 and Fig. 4 represent just one of a variety of different example magnetically responsive elements detailed in the document WO 2011/030249 A1, and the reader is referred to the aforementioned document for more examples suitable for use in accordance with embodiments of the present invention.

In accordance with a second approach described in US 20200397320 A1 a magnetically responsive element may be provided which comprises a magneto-mechanical resonator.

Fig. 5 and Fig. 6 schematically show an example magnetically responsive element 22 in accordance with this approach. The magnetically responsive element 22 comprises a magneto-mechanical resonator with two magnetic elements 507, 508 held within a casing 502.

The magnetic element 508 is suspended from a filament 506 and is thus free to perform a rotational motion about the resonator main axis. In this embodiment the further magnetic object 507 is fixed. However, in another embodiment the further magnetic element can also be suspended from a filament and thus can be free to perform a rotational motion about the resonator main axis.

At least a part of the casing 502 is flexible for allowing to transduce external pressure changes into changes of the mechanical oscillation of the magnetic object 507. Preferentially the casing comprises a deflectable membrane 515 as schematically and exemplarily shown in Fig. 5 and Fig. 6. The deflection depends on pressure exerted on the casing 502 of the magnetically responsive element 22 and changes the inter-sphere distance. A reduction in distance results in an increase of the resonance frequency and vice versa. In Fig. 5 and Fig. 6 the basic pressure sensor working principle can be seen. An increase in pressure deflects the membrane 515 and reduces the distance between the spheres 507, 508, resulting in an increase in resonance frequency. Fig. 5 and Fig. 6 further show a casing 502 and a filament 506 via which the magnetic sphere 508 is attached to the membrane 515. In Fig. 5 the pressure acting on the membrane and the resonance frequency are smaller than in Fig. 6.

In equilibrium, the magnets 507, 508, respectively, align with anti-parallel orientation of their magnetization. An external magnetic field pulse can be used to start a resonance rotational oscillation. The attractive force determines the resonance frequency of the oscillation, which for a spherical suspended magnet is given by

$$\omega_0 = \sqrt{\frac{5 M_S B}{2 \rho r^2}}$$

where $M_S$ is the saturation magnetization of the magnetic material, $p$ is its density, $r$ is the sphere diameter, and $B$ is the field created by the fixed magnet (i.e. sphere 507). It can be approximated as a dipole field

$$B(r) = \frac{\mu_0}{4\pi}\left(\frac{3r(m\cdot r)}{r^5} - \frac{m}{r^3}\right)$$

where m is the magnetic moment of the magnet.

In the presence of the external oscillating magnetic field, the field variation generated by the consequently oscillating magnetic element 508 can be detected via the induced voltage in one or several receiver coils of the receiver coil arrangement 26. The time trace of the detected signal can be Fourier-transformed to obtain the frequency spectrum which enables determination of the resonance frequency.

Due to the low resonance frequencies of a few kHz, the magnetic fields are not shielded by metal and thus all non-ferromagnetic metals can be used as structural or coating materials.

The basic magneto-mechanical oscillator of the magnetically responsive element 22 contains two magnetic elements 507, 508, wherein, in equilibrium, the magnetic elements align with anti-parallel magnetization arising from intrinsic magnetization of at least one of the two spheres 507, 508.

An external field pulse can be used to start a rotational oscillation of the suspended sphere 508 about the main axis of the resonator, wherein the other sphere 507, i.e. the further magnetic object, is fixed. If in another embodiment also the other sphere is suspended in free space and can perform a rotational oscillation, both spheres can perform a resonance counter-oscillation.

As the energy is stored mainly in the magnetic field, it is relatively easy to attain a high quality factor. High oscillation amplitudes are also easily possible. The thin filament is not subjected to strong wear. The resonance can be easily changed by changing the magnetic field by a mechanical movement of magnets relative to each other. This is also easily matched to a pressure change (using the right compliance and right shape materials as discussed below), so a quite high frequency change can be reached.

In the embodiment with a fixed sphere, the fixed sphere might have a diameter of 620 μm, whereas the oscillating sphere 508 might have a diameter of 500 μm. The magnetic moment of the oscillating sphere 508 might be m≈70 μAm2, the base frequency might be f0≈2 kHz, and the quality factor might be roughly Q≈500. SNR depends on distance between a) the receiver coils 26 used for reading out the resonance frequency and b) the magnetically responsive element 22, as well as coil parameters.

There are several ways how to attach the thread to the movable magnetic object. For instance, a through hole attachment can be used. In this case a hole is drilled through the center of gravity and roughly perpendicular to the magnetization of the sphere(s). Although the magnet material is hard and brittle, there are several methods to drill the holes, like pulsed laser or electrical discharge machining (EDM). The thread is run through the hole and glued in place. Running through is best done using a vacuum suction process. Several glue types can be used. Economic are light curing glues. They should have a low viscosity to fill the hole with the treads simply by capillary force. Alternatively, or in addition the thread can be fixed to the magnetic object by mechanical means. E.g., by having a knot in the thread or some other thick portion in the tread like a glue droplet or a heat generated (melted) bead. The latter is especially easily made in UHMWPE fibers. This attachment method reduces the magnetic dipole moment only by a small fraction and therefore retrains good signal. The shape of the magnetic object is not much altered which may be important in the case of spheres.

Also, a clamp attachment can be used. In this case the magnetic object is split in at least two components. Preferably a split plane is generated orthogonal to the magnetization and parallel to the thread attachment direction. The thread, i.e., the filament, is placed on this plane. Precise alignment is not necessary. The second magnetic part is placed on top. The magnetic parts are usually held together by magnetic forces. Finally, glue is applied to secure everything in place. Preferred glue types are the same as in the through hole attachment process. In addition, it is possible to grind a groove in one or both of the magnetic objects to reduce the overall gap between the magnetic objects. This method produces results almost as good as the through hole method but requires no special equipment for the manufacturing. Usually, the magnetic sub-objects are not made by splitting a single full magnetic object, but by grinding down two (identical) magnetic objects. The downside is that this process is more wasteful as two initial objects are used, and it may be also somewhat more labor intense.

In the following, a configuration of the receiver coil arrangement 26 and the pressure readout module 48 suitable for use with the example magnetically responsive element of Fig. 5 and Fig. 6 will be explained.

In the illustrated example, one field generator coil 24 is provided which is connected to a driver module 42 of processing unit 32 via a digital-to-analog converter 1506 (DAC) and an audio amplifier 1502 for generating the magnetic field across the volume 28. In the illustrated example, a single receive coil 26 is further provided also connected to a pressure readout module 48 of the processing unit 32 via a low noise amplifier 1505 and an analog-to-digital converter 1508 (ADC) for reading out the pressure from measurement of resonance frequency. The processing unit in this example is shown connected to a display computer 1509.

The driver module 42 controls generation of a magnetic field which comprises transmit pulses that are amplified using the audio amplifier 1502 and then passed to the field generator coil 24 which can also be named excitation coil. In this implementation, a separate receive coil 26 is employed, which is decoupled from the transmit coil 24 using two additional decoupling coils 1510 which are not shown in Fig. 7 for clarity reasons. The receive signal is fed into the low-noise amplifier 1505 and passed to the ADC 1508 and the pressure readout module 48, where a time trace of typically 1/20 of a second is sampled at a rate of about 20 kS/s. The induced voltage in the receive coil 26 is influenced due to sphere oscillation in the magnetically responsive element 22.

For further details, the reader is referred to document US 2020/0397320 A1.

It is also note that the example of Fig. 5, Fig. 6 and Fig. 7 represents just one example implementation of the general approach outlined in US 2020/0397320 A1, and more generally embodiments of the present invention can employ use of any of the example pressure sensing resonator solutions outlined in the aforementioned document.

Thus, from the above example implementations of the magnetically responsive elements, some more general principles of operation can be stated.

In some embodiments, each of the one or more magnetically responsive elements 22 is deformable and exhibiting a deformation which varies as a function of applied pressure; and wherein the magnetic signal generated by each element varies as a function of the deformation state of the element. In the example of Fig. 3, the changing pressure deforms the shape of the element 22 which changes a magnetization and/or changes a resonance frequency of the element. In the example of Fig. 5 and Fig. 6, the changing pressure deforms the outer casing 502 which changes the resonant frequency of the oscillating sphere 508.

In some embodiments, the driver module 42 is adapted to drive generation of an oscillating magnetic field. However, this is not essential. For example, the embodiment in Fig. 3 can operate using a static magnetic field.

As has been discussed, in some embodiments, each of the magnetically responsive elements is adapted to mechanically oscillate at a respective characteristic resonant frequency in response to exposure to the oscillating magnetic field, said oscillation generating the magnetic signal for the respective element.

In some embodiments, each of the magnetically responsive elements is such that said characteristic resonant frequency varies within a respective characteristic resonant frequency band as a function of deformation of the element.

In some embodiments, the device 20 comprises a plurality of the magnetically responsive elements 22, and wherein each element 22 is adapted to oscillate with a characteristic resonant frequency which is different from every other element and which varies within a characteristic resonant frequency band/range which is different from every other element.

This allows for distinguishing magnetic signals originating from different of the magnetically responsive elements. The bands/ranges may preferably be non-overlapping.

For each receive coil signal, the signal in the frequency domain can be analyzed to detect variations as a function of time within each known frequency band, and from this, pressure can be detected for each separate magnetically responsive element based on a respective transfer function or lookup table mapping measured frequency to pressure measurement.

The steps to achieve this would thus be as follows: measure the voltage signal from the receive coil; transform the signal from the time domain to frequency domain, e.g. with a Fourier transform; detect each frequency component; identify the magnetically responsive element to which each frequency component corresponds by lookup table (assuming non-overlapping resonant frequency ranges); identify by lookup table or transfer function a pressure reading corresponding to each detected frequency component.

In some embodiments, the tissue pressure sensor 4 comprises a plurality of the magnetically responsive elements 22, arranged to be sensitive to pressure at different locations across a surface of the body part of the user during use, and wherein the readout module 48 is adapted to detect a pressure reading for each magnetically responsive element 22. The readout module may be adapted to compute a pressure distribution across a surface of the body part based on a known spatial arrangement of the magnetically responsive elements relative to the cuff.

In some embodiments, the receiver coil arrangement 26 comprises a plurality of receiver coils. The readout module 48 or the one or more processors 34 may be adapted to determine a positioning of each of the one or more magnetically responsive elements 22 within the volume 28 based on analysis of a signal strength of the magnetic signal from each element 22 sensed at each receive coil.

There are at least two ways to employ the magnetically responsive elements 22 as outlined above for advantage in a blood pressure measurement cuff.

In one approach, one or more of the elements 22 may be utilized to facilitate the tissue pressure sensor 4, as illustrated in Fig. 2, and particular implementations according to this approach will be discussed in more detail to follow.

Additionally, or alternatively, in a second approach, one or more of the magnetically responsive elements can be used to measure air pressure inside the inflatable bladder 2 of the cuff 14. This provides the advantage of wireless pressure readout, which simplifies the device structurally. It provides the further advantage of improved accuracy since there is no pressure loss along the fluid tube which is normally used to fluidically connect the bladder to a pressure transducer.

For implementation as part of a tissue pressure sensor 4, in some embodiments, the magnetically responsive elements 22 may be provided encapsulated within a fluid-filled pocket or bag or pad. By way of example, the tissue pressure sensor might comprise at least one sensor pad which may contain a pressure transmitting hydraulic substance, e.g., a water-based gel, silicone oil or anything similar. In some embodiments, each of the one or more magnetically responsive elements may be provided encapsulated in a separate respective fluid-filled pocket or bag or pad.

In some embodiments, a single magnetically responsive element 22 may be provided.

More advantageously, in some embodiments, a plurality of the magnetically responsive elements 22 may be provided. A plurality of the elements 22 may be provided in a formation wherein the elements are arranged to be sensitive to pressure at different locations across a surface of the body part of the user during use. The readout module may be adapted to detect a pressure reading for each magnetically responsive element. Techniques for separating signal components received from multiple different magnetically responsive elements have already been described above.

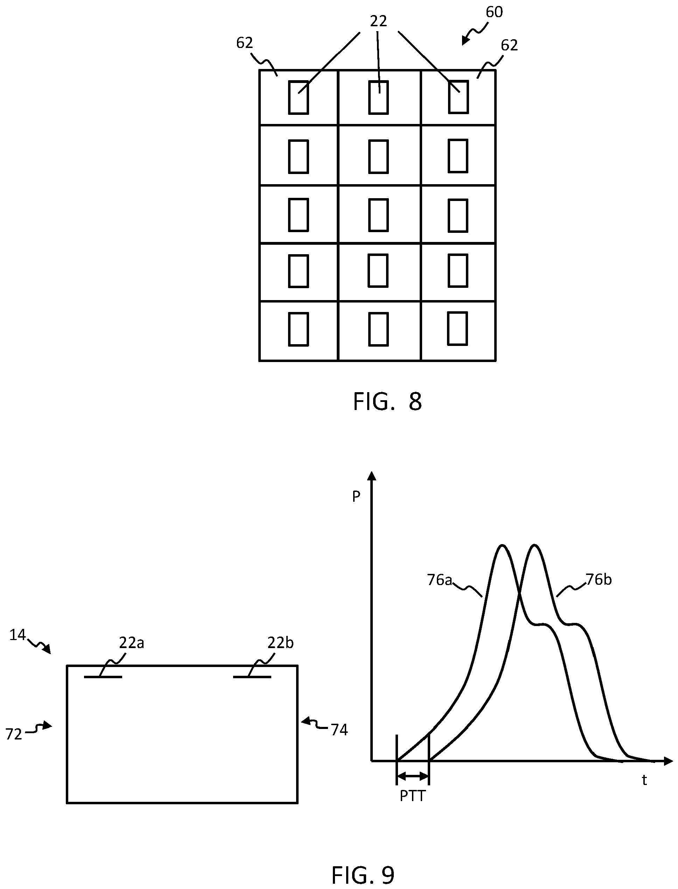

An example configuration of the magnetically responsive elements 22 according to one or more embodiments is shown in Fig. 8. This shows a sensing arrangement 60 which might be comprised by a tissue pressure sensor 4 or a cuff pressure sensor according to one or more embodiments. The arrangement 60 comprises an array of magnetically responsive elements 22. In this example, the array is a 2D array. However, a ID array might also be considered. In this example, a regular grid array is shown, but in other examples a differently shaped array could be provided, e.g. curved, circular, or annular. In this example, each magnetically responsive element 22 is provided disposed in a separate respective fluid pocket or pouch 62 which facilitates an even and smooth transfer of pressure onto the magnetically responsive element. As discussed above, each element is adapted to generate a magnetic response to an applied stimulus field which varies as a function of pressure to which the element is exposed. Pressure applied on an outside of given fluid pocket 62 will be transferred into a change in fluid pressure inside the pocket, which in turn will be exerted as a change in applied pressure on an outside of the magnetically responsive element 22. This then leads to the change in magnetic response characteristics of the element which is detectable in the magnetic signals received from each element at the receiver coil arrangement 26 and the readout module 48.

Using the array formation of magnetically responsive elements 22 allows for a spatial pressure distribution to be derived by obtaining a pressure signal for each respective element 22. This thus allows for set of spatially distributed pressure readings. This might allow in some examples for determining a spatial distribution of pressure across an area of the user's body part. In this case, the readout module is adapted to compute a pressure distribution across a surface of the body part based on a known spatial arrangement of the magnetically responsive elements relative to the cuff.

In some embodiments, the plurality of magnetically responsive elements 22 can be used to improve signal quality of a pressure measurement, instead of, or in addition to, deriving a spatial pressure distribution. In this case, the plurality of pressure signals received from the plurality of magnetically responsive elements 22 may either be compared to identify a strongest signal, or averaged to obtain a less error- or noise- sensitive signal.

For instance, by way of one example, the device 20 may include a plurality of the magnetically responsive elements 22 arranged for sensing pressure at different locations across a surface of the body part when the cuff is worn, and wherein the processing unit 32 is configured to perform the following steps: detect the magnetic signal associated with each different magnetically responsive element 22; determine a signal strength associated with each magnetic signal; identify the signal with the highest signal strength; and compute a pressure reading based on the identified magnetic signal. In some embodiments, the processing unit 32 may further be configured to forward the identified pressure reading to a biological parameter measurement module.

In some embodiments, identification of the strongest-responding magnetically responsive element 22 could be performed as an initialization step. This could be achieved by controlling the field generator coils 24 to generate a test magnetic signal, for example a pulse signal, and measuring the magnetic response signal generated by each magnetically responsive element. As discussed above, in some embodiments, each element is configured to generate a magnetic response signal within a unique magnetic frequency range, and thus signals from different magnetically responsive elements may be separated based on frequency analysis applied to the composite magnetic signal measured at the receiver coil arrangement 26. Then, for each separated signal, its signal strength (e.g. measured voltage amplitude) might be determined, and these strengths compared to identify the strongest signal. Additionally, or alternatively, a signal strength may correspond to a signal to noise ratio for the signal.

Additionally or alternatively, the device 20 may include a plurality of the magnetically responsive elements 22 arranged for sensing pressure at different locations across a surface of the body part when the cuff is worn, and wherein the processing unit 32 is adapted to perform the following sequence of steps: detect the magnetic signal associated with each different element 22; determine a signal strength associated with each magnetic signal; determine a pressure measurement for each element based on the respective magnetic signal; and generate a weighted average pressure reading, wherein the weightings are determined based on the signal strength of the magnetic signal associated with each respective pressure measurement. In some embodiments, the processing unit 32 may further be configured to forward the weighted average pressure reading to a biological parameter measurement module.

In some embodiments, an arrangement of multiple of the magnetically responsive elements can be used to provide information about blood flow or blood pulse characteristics.

An example is schematically shown in Fig. 9.

Here, the device comprises a plurality of the magnetically responsive elements 22, arranged to be sensitive to pressure at different locations across a surface of the body part of the user during use, and wherein, when the cuff 14 is wrapped around the body part, the body part extends through the cuff between a proximal 72 and distal 74 end of the cuff, and wherein the plurality of magnetically responsive elements 22 includes a first element 22a closer to the proximal end 72, and a second element 22b closer to the distal end 74. In Fig. 9, the cuff 14 is shown comprising just a single first element 22a and a single second element 22b. However, more than just these two elements might be provided in further embodiments. For example, these elements could be provided in addition to the arrangement 60 of elements described above in relation to Fig. 9, or similar.

Each of the first 22a and second 22b elements may be provided as respective parts of a tissue pressure sensor 4, arranged for sensing pressure between the cuff 14 and the body part of the patient when the cuff is worn.

Using the first 22a and second 22b magnetically responsive elements of Fig. 4, information about for example pulse transit time can be derived. This can be derived for instance as the time difference of the synchronously detected tissue pressures measured at each of the first 22a and second 22b elements.

Fig. 9 (right) illustrates a first pressure signal 76a (y-axis) as a function of time (x-axis) measured from the first magnetically responsive element 22a, and a second pressure signal 76b as a function of time measured from the second magnetically responsive element 22b.

A time offset between the two signals can be measured by comparing the time-stamps corresponding to a similar fiducial point in each signal, e.g. comparing a time stamp of a point at a foot of one signal 76a against that of the other signal 76b. This is illustrated schematically in Fig. 9. This time offset can be associated with a measurement of pulse transit time (PTT). Instead of the foot of the signal, a different fiducial point could be used such as peak of the signal for instance. As another example, instead of comparing time stamps of fiducial points, cross-correlation between the first 76a and second 76b signals could be performed.

As mentioned above, by providing a plurality of the magnetically responsive elements in a spatially distributed arrangement, it is possible to determine a pressure distribution across the area of the user's tissue covered by the arrangement.

Another functionality made possible by inclusion of a plurality of magnetically responsive elements is to use signal strengths of the multiple magnetic response signals generated by the multiple elements to determine a spatial arrangement of the elements relative to the body part. This can allow for determining information about geometry or size of the body part, e.g., arm circumference.

For this set of embodiments, the receiver coil arrangement 26 may comprise a plurality of receiver coils, each operable to sample the magnetic signals generated by the plurality of magnetically responsive elements 22. The processor unit 32 may be adapted to determine a positioning of each of the one or more magnetically responsive elements 22 within the volume 28 based on analysis of a signal strength of the magnetic signal received from each element as sensed at each receive coil. For example, this may be done through standard triangulation techniques. To state this another way, the magnetic signal generated by each of the plurality of magnetically responsive elements (responsive to the applied field) is measured separately by a plurality (preferably at least three) receiver coils which are disposed at different spatial locations relative to the magnetically responsive element. By measuring a signal strength (e.g., signal amplitude) at each of the plurality of coils, mapping this to an estimated distance of the element from the receive coil, and then triangulating the distances, the position of the element relative to the coils can be determined.

In practice, localization of an individual magnetically responsive element can be achieved by comparing the set of signals measured from the set of receiver coils against a plurality of reference signal sets, where each reference signal set corresponds to a known position of the magnetically responsive element. Each reference signal set is thus the expected signal set if the magnetically responsive element is located at a particular defined location. By finding the best fit between the measured set of signals and the reference signal sets the most likely location for the magnetically responsive element can be identified.

In preferred examples, at least five receiver coils may be included to facilitate this functionality, and more preferably at least six receiver coils. This allows for determining the location with all spatial freedoms (three positional degrees of freedom, three rotational degrees of freedom).

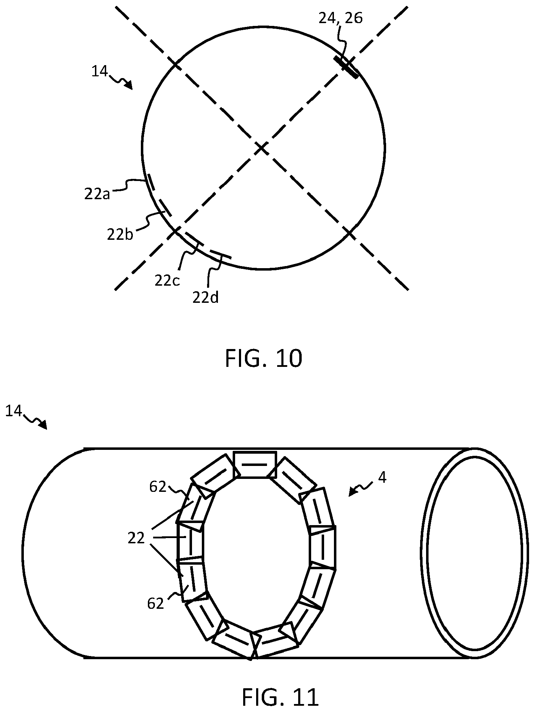

An example of this is illustrated in Fig. 10 which schematically illustrates a cross-sectional view of a cuff 14, shows how arm circumference could be measured by using an arrangement of circumferentially distributed magnetically responsive elements 22. In particular, the cuff in this example includes a series of magnetically responsive elements 22a-22d arranged in a row along a circumferential dimension of the cuff. When the cuff 14 is being worn around the arm of a user, the set of magnetically responsive elements thus form an arcuate formation. Arranged at a circumferentially displaced position of the cuff is a coil arrangement including one or more field generator coils 24 and a receiver coil arrangement 26 comprising one or more field receiver coils. Ideally this is positioned approximately opposite to the arrangement of magnetically responsive elements 22a-22d, but in practice may be displaced circumferentially slightly offset from opposite, depending upon the particular circumference of the user's arm. The circumferential displacement of the coils 24, 26 and the magnetically responsive elements 22-22d can be defined so that approximate opposite placement is achieved for an average arm circumference based on population figures. By measuring at the receiver coil arrangement 26 the signal strength of the magnetic signal generated by each magnetically responsive element 22a-22d (individually resolved for example based on frequency in the manner already described above), then an approximate geometric positioning of the arcuate array of elements 22a-22d relative to the coils 24, 26 can be determined based on a best-fit procedure in which the measured set of signals are fit to a most likely positioning based on reference signal data comprising sets of signal measurements corresponding to different known positions. From the determined geometric positioning, a circumference of the arm can be determined.

In a simpler case, the fitting operation may simply find a most likely value for the arm circumference based on the total set of measured signals for all of the magnetically responsive elements. Here, reference data may be used which associates each of a set of different possible arm circumferences with corresponding expected sets of signal measurements. In some examples, regression fitting may be used for example. In some examples, a transfer function may be defined using the reference data which maps the measured magnetic signal strength detected at the coils for the plurality of magnetically responsive elements 22a-22d to a corresponding estimated arm circumference.

Another potential advantage of providing a spatially distributed arrangement of multiple of the magnetically responsive elements 22 is that sensitivity of the pressure measurements to the exact placement of the cuff relative to the body can be reduced.

By way of example, Fig. 11 shows an example in which sensitivity of measurements to rotational positioning of the cuff relative to the body part can be reduced. This is most particularly applicable for the case where the magnetically responsive elements 22 are utilized for incorporation in the tissue pressure sensor 4. The tissue pressure sensor should be positioned during use above the artery for which blood pressure is to be measured. This imposes a constraint on the rotational positioning of the cuff 14, so that the tissue pressure sensor can be correctly located relative to the artery when the cuff is worn.

As schematically indicated in Fig. 11, in accordance with one or more embodiments of the present invention, the tissue pressure sensor 4 may be provided comprising a plurality of the magnetically responsive elements 22, arranged to be sensitive to pressure at different locations across a surface of the body part of the user during use, and wherein the plurality of magnetically responsive elements 22 includes an annular or arcuate array of elements arranged, when the cuff 14 is wrapped around the body part, such that the array extends around the whole or part of the circumference of the body part, i.e. the inner circumference of the cuff.

As schematically illustrated in Fig. 11, optionally each of the magnetically responsive elements 22 in the array may be provided disposed in a respective fluid-filled pocket or chamber 62.

This set of embodiments enables reduced rotational sensitivity. By way of example, an operation might be performed to identify the one of the plurality of magnetically responsive elements whose pressure signal strength is strongest. This element can then be assumed to be the element closest to the artery. The pressure signals derived from just this element might then be selected for use in deriving one or more biological parameters. For example, the pressure signals from this element might be forwarded to a biological parameter measurement module.

Thus, the magnetically responsive element having the best signal quality can be automatically selected for measurement parameter computation. Application of the cuff is thus made independent of angular rotation. This eases workflow and does not require palpation of the brachial artery.

In some embodiments, the processing unit may be adapted to determine an orientation state of the cuff based on the signals from the plurality of magnetically responsive elements 22. This can be achieved based on defining a rigid model of the arm and wherein the rigid model associates different possible orientations of the cuff 14 with different possible sets of magnetic signals measured at the receiver coil arrangement 26, received from the array of magnetically responsive elements. In a simple example, an orientational position can be determined by iterating through each possible orientation in the model to identify the orientation for which the associated magnetic signals in the model best match the measured set of magnetic signals.

To facilitate this embodiment, at least the receiver coil arrangement might be integrated in a separate unit from the main cuff and attachable by a user to the main cuff after the cuff is fitted to the patient, and wherein, in use, the receiver coil arrangement is attached by a user at a pre-defined orientation position around the body part. This way, the receiver coil arrangement defines a known anchor position in orientational space relative to which the orientation position of the cuff can be determined. For example, in some embodiments, the receiver coil arrangement might be integrated in a sheath or patch which can removably adhere to an outside of the cuff, e.g., through a hook and loop (Velcro) fastener arrangement.

In some embodiments, automated correction or calibration may be performed to account for the cuff height relative to the heart height. As is known in clinical practice, due to hydrostatic effects, measured blood pressure changes as a function of the height on the body at which it is measured. Typically, a standard blood pressure measurement refers to blood pressure at the height of the heart. If, instead, the measurement is taken at a different location, the height disparity between the heart and the cuff can be compensated in the blood pressure measurement, P, using blood density, $\rho$, and the gravitational constant, $g$, according to the equation $\Delta P = \rho g \Delta h$, where $\Delta P$ is the difference in the blood pressure measurement compared to a measurement at the heart height, $g$=9.81 is the gravitational constant and $\Delta h$ is the difference in height between the cuff location when taking the measurement and the heart height. This $\Delta P$ value can simply be added or subtracted as appropriate from the obtained blood pressure measurement to obtain a compensated measurement.

In some embodiments of the present invention, it is proposed to provide a secondary unit for attachment to the body at a location coinciding in height with a height of the heart and the secondary unit incorporating a further one or more magnetically responsive elements. By measuring signal strength of magnetic signals received at the receiver coil arrangement in the cuff from the secondary unit, an approximate height of the cuff relative to the heart height can be measured. The secondary unit may for example be a patch which is adhered removably to the patient's chest, or a chest band which is worn around the chest. This allows for example for the system to account automatically for posture changes in the subject which alter the height of the cuff relative to the heart.

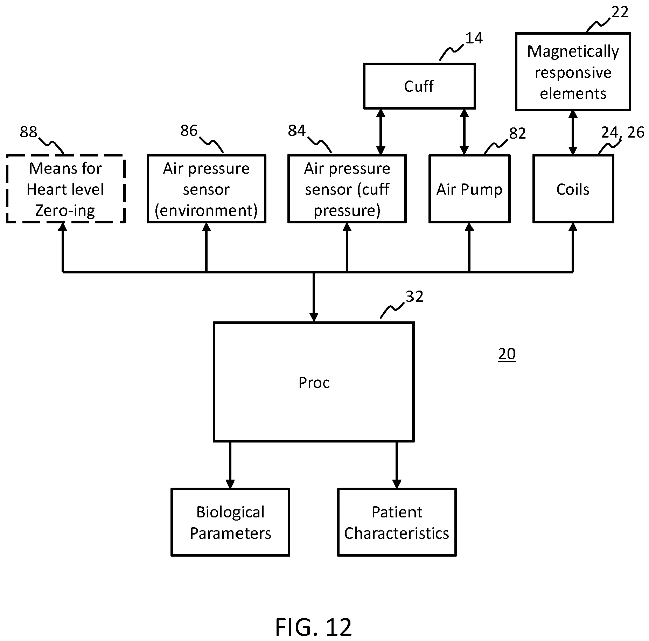

Fig. 12 outlines in block diagram form an illustration of the components of a device and associated system according to one or more particular embodiments. It will be appreciated that not all features of this particular implementation are essential to the inventive concept and are described to aid understanding and to provide an example implementation.

The illustrated device 20 comprises a measurement cuff 14, for example as previously discussed with reference to Fig. 2. The cuff 14 includes an inflatable bladder for applying a varying pressure to the body part of the user when the cuff is worn. The inflatable bladder of the cuff is fluidly connected to an air pump 82 for changing the air pressure in the bladder, and fluidly connected to an air pressure sensor 84 for sensing a pressure inside the inflatable bladder of the cuff 14. The device further comprises a set of coils which includes one or more field generator coils 24 for generating a magnetic field across a volume, wherein the one or more magnetically responsive elements are located within said volume; and further includes a receiver coil arrangement 26 for sampling said magnetic field across a sampling space within the volume.

It is noted that although in the example embodiment shown in Fig. 2, the coils are provided integrated in the cuff 14 in other examples, the coils could be provided in a separate unit which for example couples to an outside surface of the cuff once it is applied, or which is otherwise structurally separate from the cuff.

The device 20 further includes a set of one or more of the magnetically responsive elements 22 already discussed, arranged so as to be in pressure coupling arrangement with the user's body part when the cuff 14 is worn, so that changes in relative pressure between the cuff and the body part during use are coupled to a surface of each magnetically responsive element. Each of the one or more magnetically responsive elements is adapted to be magnetically stimulated upon exposure to a magnetic field and to generate a magnetic signal responsive thereto, and wherein the magnetically responsive element is such that said magnetic signal varies as a function of applied pressure on the magnetically responsive element.

The device may further include an air pressure sensor 86 for sensing an environmental (ambient) air pressure of the air surrounding the cuff. This allows for calibrating measured cuff pressure measurements and tissue pressure measurements. For example, the pressure measurements obtained from the magnetically responsive elements depend in part on the atmospheric pressure. Thus, by measuring atmospheric pressure using the dedicated sensor 86, a true value for pressure can be computed by subtracting atmospheric pressure from the pressure measurement obtained using the tissue pressure sensor or the cuff pressure sensor 84.

The device may further include a means 88 for performing heart-level calibration or correction. This optional feature has already been described above. In particular, it is proposed to provide a separate unit integrating one or more further magnetically responsive elements and which is adapted to be attached to the subject's body at the level of the heart. The height of this unit can be established based on signal strengths of magnetic signals received at the receiver coil arrangement 26 from the magnetically responsive elements integrated in the separate unit. The difference in height between the heart and the cuff can then be taken into account in calibrating blood pressure measurements as described previously.

The device 20 further includes a processing unit 32 which is configured to control the generation of the magnetic field(s) with the generator coils 24 and control the measurement of the magnetic response signals at the receiver coils 26 generated by the magnetically responsive elements 22. For example, the processing unit 32 may comprise a driver module for driving the generator coils to generate the magnetic field and a pressure readout module arranged to detect applied pressure at each magnetically responsive element based on sensing the magnetic signals from the elements within the sampled magnetic field.

In some embodiments, the processing unit 32 may be further adapted to compute one or more biological parameters based on the obtained measurements, and/or to compute one or more patient characteristics, such as geometry or size of the body part, based on the obtained measurements.

In some embodiments, the processing unit 32 may be adapted to communicate with a datastore which stores reference values for one or more biological parameters or patient characteristics. The processing unit 32 may be adapted to compare one or more derived biological parameters or patient characteristics for the patient with the reference values. The datastore may be a cloud-based datastore, and the processing unit may communicate via an internet connection.

Embodiments of the invention described above employ a processing unit. The processing unit may in general comprise a single processor or a plurality of processors. It may be located in a single containing device, structure or unit, or it may be distributed between a plurality of different devices, structures or units. Reference therefore to the processing unit being adapted or configured to perform a particular step or task may correspond to that step or task being performed by any one or more of a plurality of processing components, either alone or in combination. The skilled person will understand how such a distributed processing unit can be implemented. The processing unit includes a communication module or input/output for receiving data and outputting data to further components.

The one or more processors of the processing unit can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

**Claims**

1. A device (20) for use in blood pressure measurement comprising:

   a cuff (14) for wrapping around a part of a body of a user, and wherein the cuff includes an actuator in the form of an inflatable bladder for use in changing a pressure applied to the body part by the cuff, and
   a cuff pressure sensor arranged for sensing a gas pressure inside the inflatable bladder and/or a tissue pressure sensor (4) arranged for sensing a pressure between a surface of the body part and the cuff, and
   wherein the cuff pressure sensor and/or the tissue pressure sensor (4) comprises:

      one or more magnetically responsive elements (22) arranged so as to be in pressure coupling arrangement with the user's body part when the cuff is worn, so that changes in relative pressure between the cuff and the body part during use are coupled to each magnetically responsive element;
      wherein each of the one or more magnetically responsive elements is adapted to be magnetically stimulated upon exposure to a magnetic field and to generate a magnetic signal responsive thereto, and wherein the magnetically responsive element is such that said magnetic signal varies as a function of applied pressure on the magnetically responsive element;

      one or more field generator coils (24) for generating a magnetic field across a volume, wherein the one or more magnetically responsive elements are located within said volume;
a receiver coil arrangement (26) comprising one or more field receiver coils for sampling said magnetic field across a sampling space within the volume; and
a processing unit comprising one or more processors, the processing unit including:

   a driver module (42) for driving the generator coils to generate the magnetic field, and
   a pressure readout module (48) arranged to detect applied pressure at each magnetically responsive element based on sensing the magnetic signals from the elements within the sampled magnetic field.

2. The device of claim 1,

   each of the one or more magnetically responsive elements comprising a magnetic or magnetizable body which is deformable and exhibits a deformation which varies as a function of applied pressure; and
   wherein the magnetic signal generated by each body varies as a function of the deformation state of the body.

3. The device of claim 1 or 2, wherein the driver module is adapted to drive generation of an oscillating magnetic field, and wherein each of the magnetically responsive elements comprises at least a part which is adapted to mechanically oscillate at a respective characteristic resonant frequency in response to exposure to the oscillating magnetic field, said oscillation generating the magnetic signal for the respective element.

4. The device of claim 3, wherein each of the magnetically responsive elements is such that said characteristic resonant frequency varies within a respective characteristic resonant frequency band as a function of applied pressure on the element, and optionally wherein

   the device comprises a plurality of the magnetically responsive elements; and
   each magnetically responsive element is adapted to oscillate with a characteristic resonant frequency which is different from every other element; and which varies within a characteristic resonant frequency band which is different from every other element.

5. The device of any of claims 1-4, wherein the tissue pressure sensor comprises a plurality of the magnetically responsive elements, arranged to be sensitive to pressure at different locations across a surface of the body part of the user during use; and

wherein the readout module is adapted to detect a pressure reading for each magnetically responsive element.

6. The device of any of claims 1-5, wherein the receiver coil arrangement comprises a plurality of receiver coils, and wherein the processor is adapted to determine a positioning of each of the one or more magnetically responsive elements within the volume based on analysis of a signal strength of the magnetic signal from each element sensed at each receiver coil.

7. The device of claim 6,

   wherein the device includes a plurality of the magnetically responsive elements arranged for sensing pressure at different locations across a surface of the body part when the cuff is worn; and

   wherein the processor is adapted to determine geometry or shape information related to the body part based on the detected positioning of the plurality of elements.

8. The device of any preceding claim,

   wherein the device includes a plurality of the magnetically responsive elements arranged for sensing pressure at different locations across a surface of the body part when the cuff is worn; wherein the processing unit is configured to:

      detect the magnetic signal associated with each different element;
      determine a signal strength associated with each magnetic signal;
      identify the signal with the highest signal strength;
      compute a pressure reading based on the identified magnetic signal; and
      forward the identified pressure reading to a biological parameter measurement module.

9. The device of any preceding claim,

   wherein the device includes a plurality of the magnetically responsive elements arranged for sensing pressure at different locations across a surface of the body part when the cuff is worn; wherein the processing unit is adapted to:

      detect the magnetic signal associated with each different element;
      determine a signal strength associated with each magnetic signal;
      determine a pressure measurement for each element based on the respective magnetic signal;
      generate a weighted average pressure reading, wherein the weightings are determined based on the signal strength of the magnetic signal associated with each respective pressure measurement;
      forward the weighted average pressure reading to a biological parameter measurement module.

10. The device of any of claims 1-9,

    wherein the tissue pressure sensor comprises a plurality of the magnetically responsive elements, arranged to be sensitive to pressure at different locations across a surface of the body part of the user during use
    wherein, when the cuff is wrapped around the body part, the body part extends through the cuff between a distal and proximal end of the cuff, and wherein the plurality of elements includes a first element closer to the proximal end, and a second element closer to the distal end, and optionally wherein the processing unit is adapted to determine a pulse transit time based on pressure readings from the first and second elements.

11. The device of any of claims 1-10,

    wherein the tissue pressure sensor comprises a plurality of the magnetically responsive elements, arranged to be sensitive to pressure at different locations across a surface of the body part of the user during use;
    wherein the plurality of magnetically responsive elements is arranged such that, when the cuff is wrapped around the body part, the elements are at different circumferential locations around the body part.

12. The device of any of claims 1-11, wherein the plurality of elements includes an annular or arcuate array of elements arranged, when the cuff is wrapped around the body part, such that the array extends around the whole or part of the circumference of the body part.

13. The device of any of claims 1-12, wherein each magnetically responsive element is located inside a respective fluid-filled pocket.

14. The device of any of claims 1-13, wherein the one or more processors further include a biological parameter measurement module including one or more algorithms for computing one or more biological parameters based on the one or more pressure readings.

**15.** The device of any of claims 1-14, wherein the device includes a shell portion,

wherein the shell portion is arranged so as to be located between the actuator and the body part, when the cuff is wrapped around the body part wherein the shell portion is arranged to surround the body part when the cuff is wrapped around the body part; and wherein the shell portion comprises a plurality of overlapping sections configured to slide relatively to each other, permitting variation of the diameter of the shell portion responsive to application of pressure by the actuator.

FIG. 1

FIG. 2

FIG. 3

FIG. 4

FIG. 5

FIG. 6

FIG. 7

FIG. 8

FIG. 9

14

24, 26

22a

22b

22c

22d

## FIG. 10

14

62

22

62

4

## FIG. 11

FIG. 12

Europäisches
Patentamt
European
Patent Office
Office européen
des brevets

# EUROPEAN SEARCH REPORT

Application Number

EP 22 20 9785

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | EP 3 430 992 A1 (UP MED GMBH [DE]) 23 January 2019 (2019-01-23) * figures 2a, 2b, 9 * * paragraph [0057] * * paragraph [0067] * * paragraph [0084] – paragraph [0086] * * paragraph [0089] * ----- | 1–15 | INV. A61B5/022 A61B5/021 A61B5/0295 A61B5/00 |
| Y,D | US 2020/397320 A1 (GLEICH BERNHARD [DE] ET AL) 24 December 2020 (2020-12-24) * figures 11,12,20 * * paragraph [0117] * * paragraph [0125] * * paragraph [0141] – paragraph [0144] * * paragraph [0160] * * paragraph [0180] * * paragraph [0193] * * paragraph [0204] – paragraph [0205] * * paragraph [0211] * * paragraph [0234] * ----- | 1–15 | |

TECHNICAL FIELDS
SEARCHED        (IPC)

A61B

The present search report has been drawn up for all claims

| Place of search | Date of completion of the search | Examiner |
|---|---|---|
| The Hague | 12 May 2023 | Oancea, A |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
        ...................................................................
& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 22 20 9785

This annex lists the patent family members relating to the patent documents cited in the above-mentioned European search report.
The members are as contained in the European Patent Office EDP file on
The European Patent Office is in no way liable for these particulars which are merely given for the purpose of information.

12-05-2023

| Patent document cited in search report | | | Publication date | Patent family member(s) | | | Publication date |
|---|---|---|---|---|---|---|---|
| EP 3430992 | A1 | | 23-01-2019 | EP | 2953528 | A1 | 16-12-2015 |
| | | | | EP | 3430992 | A1 | 23-01-2019 |
| US 2020397320 | A1 | | 24-12-2020 | AU | 2019290959 | A1 | 11-02-2021 |
| | | | | AU | 2019451287 | A1 | 17-02-2022 |
| | | | | AU | 2019451647 | A1 | 17-02-2022 |
| | | | | BR | 112020025733 | A2 | 16-03-2021 |
| | | | | BR | 112021025765 | A2 | 03-03-2022 |
| | | | | BR | 112021026008 | A2 | 21-06-2022 |
| | | | | CA | 3104001 | A1 | 26-12-2019 |
| | | | | CA | 3144130 | A1 | 24-12-2020 |
| | | | | CA | 3144550 | A1 | 24-12-2020 |
| | | | | CN | 112107364 | A | 22-12-2020 |
| | | | | CN | 112113584 | A | 22-12-2020 |
| | | | | CN | 112384134 | A | 19-02-2021 |
| | | | | CN | 114269233 | A | 01-04-2022 |
| | | | | CN | 114269237 | A | 01-04-2022 |
| | | | | EP | 3583890 | A2 | 25-12-2019 |
| | | | | EP | 3583892 | A1 | 25-12-2019 |
| | | | | EP | 3583896 | A1 | 25-12-2019 |
| | | | | EP | 3809961 | A1 | 28-04-2021 |
| | | | | EP | 3986264 | A1 | 27-04-2022 |
| | | | | EP | 3986271 | A1 | 27-04-2022 |
| | | | | JP | 2021000419 | A | 07-01-2021 |
| | | | | JP | 2021001863 | A | 07-01-2021 |
| | | | | JP | 2021528142 | A | 21-10-2021 |
| | | | | JP | 2022546897 | A | 10-11-2022 |
| | | | | JP | 2023505402 | A | 09-02-2023 |
| | | | | US | 2020397320 | A1 | 24-12-2020 |
| | | | | US | 2020397510 | A1 | 24-12-2020 |
| | | | | US | 2020397530 | A1 | 24-12-2020 |
| | | | | US | 2020400509 | A1 | 24-12-2020 |
| | | | | US | 2021244305 | A1 | 12-08-2021 |
| | | | | WO | 2019243098 | A1 | 26-12-2019 |
| | | | | WO | 2020253977 | A1 | 24-12-2020 |
| | | | | WO | 2020253978 | A1 | 24-12-2020 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2953528 A1
- WO 201130249 A1
- US 2020397320 A1
- US 2015359442 A
- US 2015320364 A
- WO 2011030249 A1
- US 20200397320 A1